# EUROPEAN PATENT APPLICATION

(11) **EP 4 840 438 A1**
(43) Date of publication of application: **18.11.2026**
(21) Application number: 24916885.7
(22) Date of filing: 20.12.2024
(51) Int. Cl.: A61K 8/25, A61K 8/02, A61K 8/34, A61K 8/42, A61K 8/86, A61Q 19/00, C01B 33/18

(54) **SILICA COMPOSITE PARTICLES**

(30) Priority: 12.01.2024 JP 2024003122
(71) Applicant: Kao Corporation, Tokyo 103-8210 (JP)
(72) Inventor: ISHIGAMI Takaaki, Tokyo 103-8210 (JP); KINOSHITA Takafumi, Tokyo 103-8210 (JP); MAETA Yuya, Tokyo 103-8210 (JP)
(74) Representative: Hoffmann Eitle
(86) International application number: PCT/JP2024/045079
(87) International publication number: WO 2025/150383

(57) **Abstract**

Composite silica particles in which a nonvolatile component containing ceramides is supported on porous silica particles, wherein the porous silica particles have a pore volume of 0.5 mL/g or more, and a relative pore volume ratio (the ratio of the content by volume of the nonvolatile component to the pore volume of the porous silica particles) is 110% or less; [2] granulated particles containing the composite silica particles of [1]; [3] a method for producing the composite silica particles of [1], the method comprising, in this order, step 1 of obtaining a suspension and step 2 of drying the suspension; and [4] a method for producing the granulated particles of [2], the method comprising, in this order, step 1' of obtaining a suspension and step 2' of spray-drying the suspension.

## Description

### Field of the Invention

The present invention relates to composite silica particles, granulated particles of the composite silica particles, and methods for producing the same.

### Background of the Invention

Ceramides are present as components of intercellular lipids in the stratum corneum, which is the outermost layer of the skin, and play an important role in maintaining the skin's inherent function as a barrier between the body and the external environment. It is widely known that, when such ceramides are incorporated into external preparations, cosmetic preparations, or the like and applied to the skin, they are effective for improving moisturizing effects and for improving care effects such as prevention or improvement of rough skin.

As an example of a cosmetic preparation containing ceramides, JP-A 2001-158717 (Patent Literature 1) discloses a solid powder cosmetic preparation containing:
(A) 60 to 75 weight% of a powder component which contains a porous inorganic powder in an amount of 0.1 to 20 weight% based on the total composition; and
(B) 25 to 40 weight% of an oily component which contains a moisturizing component in an amount of 0.1 to 15 weight% based on the total composition,
and discloses ceramides as the moisturizing component.

### Summary of the Invention

The present invention relates to composite silica particles in which a nonvolatile component containing ceramides is supported on porous silica particles,
wherein:
the porous silica particles have a pore volume of 0.5 mL/g or more;
the nonvolatile component has a boiling point (at 1,013 hPa) of 150°C or higher; and
a relative pore volume ratio determined by the following formula (1) is 110% or less: 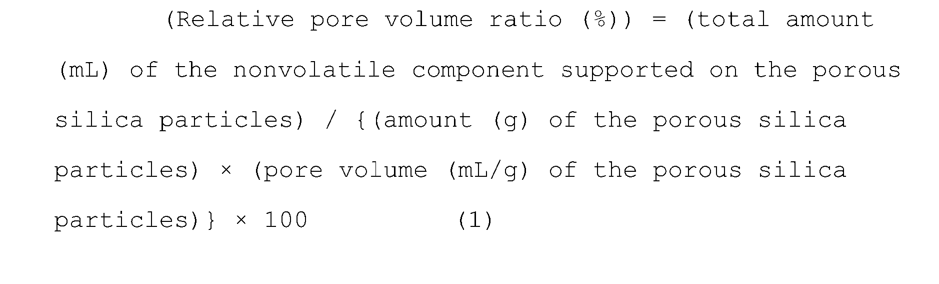 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)

### Embodiments of the Invention

Although the solid powder cosmetic preparation of Patent Literature 1 provides both a moisturizing feel and a smooth feel when used, Patent Literature 1 does not discuss the amount of moisturizing components, particularly ceramides, released into sebum, and ceramides may not be used sufficiently effectively in some cases.

The present invention relates to composite silica particles and granulated particles of the composite silica particles, each of which can readily release ceramides upon contact with oleic acid, a major component of sebum, and to methods for producing the same.

The present inventors have found that the above issue can be solved by supporting, on porous silica particles having a specified pore volume, a nonvolatile component containing ceramides in a specified amount relative to the pore volume of the porous silica particles.

The present invention relates to the following [1] to [4].
[1] Composite silica particles in which a nonvolatile component containing ceramides is supported on porous silica particles,
   wherein:
   the porous silica particles have a pore volume of 0.5 mL/g or more;
   the nonvolatile component has a boiling point (at 1,013 hPa) of 150°C or higher; and
   a relative pore volume ratio determined by the following formula (1) is 110% or less: 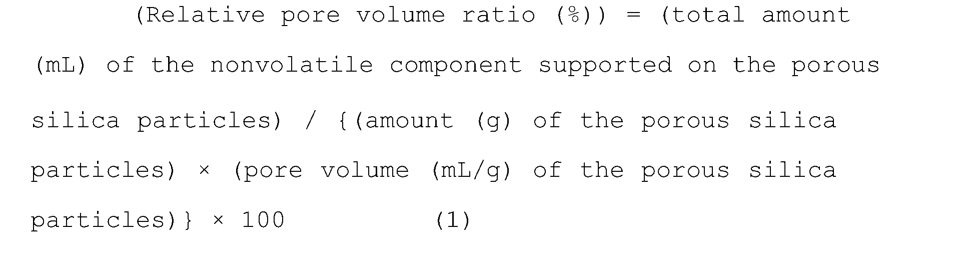 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)
[2] Granulated particles containing the composite silica particles according to [1].
[3] A method for producing the composite silica particles according to [1], comprising the following steps 1 and 2 in this order:
   step 1: a step of mixing an emulsion or a solution containing the nonvolatile component containing ceramides with the porous silica particles to obtain a suspension; and
   step 2: a step of drying the suspension obtained in step 1.
[4] A method for producing the granulated particles according to [2], comprising the following steps 1' and 2' in this order:
   step 1': a step of mixing an emulsion or a solution containing the nonvolatile component containing ceramides, the porous silica particles, and a water-soluble polymer to obtain a suspension; and
   step 2': a step of spray-drying the suspension obtained in step 1'.

The present invention can provide composite silica particles and granulated particles containing the composite silica particles and a water-soluble polymer, each of which can readily release ceramides upon contact with oleic acid, a major component of sebum, and methods for producing the same.

### [Composite silica particles]

The composite silica particles of the present invention are composite silica particles in which a nonvolatile component containing ceramides is supported on porous silica particles,
wherein:
the porous silica particles have a pore volume of 0.5 mL/g or more;
the nonvolatile component has a boiling point (at normal pressure of 1,013 hPa) of 150°C or higher; and
a relative pore volume ratio determined by the following formula (1) is 110% or less: 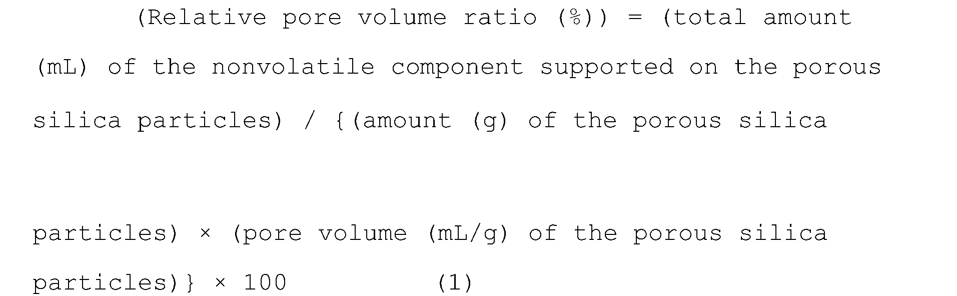 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)

The composite silica particles of the present invention can readily release the ceramides contained in the pores of the porous silica particles upon contact with oleic acid, a major component of sebum. The reason for this is not clear; however, it is considered to be as follows.

In the composite silica particles of the present invention, the nonvolatile component containing ceramides is supported in the pores of the porous silica particles, and it is considered that this helps maintain the ceramides in an amorphous state. It is considered that, since the ceramides are in an amorphous state, they dissolve in oleic acid more quickly than they would in a crystalline state. It is therefore considered that the composite silica particles of the present invention increase the amount of ceramides released upon contact with oleic acid present on the skin.

Furthermore, in the composite silica particles of the present invention, since the porous silica particles have a pore volume of 0.5 mL/g or more, a sufficient amount of the nonvolatile component containing ceramides can be supported in the pores.

In addition, it is considered that, in the composite silica particles of the present invention, by setting the relative pore volume ratio (the ratio of the content by volume of the nonvolatile component to the pore volume of the porous silica particles) to 110% or less, the amount of ceramides crystallizing outside the pores of the porous silica particles can be suppressed, thereby preventing plugging of the pore openings. As a result, it is considered that a decrease in the amount of ceramides released upon contact with oleic acid, a major component of sebum, can be suppressed. It should be noted that a relative pore volume ratio of 110% indicates that, when the amount of the nonvolatile component present inside the pores of the porous silica particles is taken as 100%, at least an additional 10% of the nonvolatile component is present outside the pores while adhering to the surfaces of the porous silica particles.

As used herein, the relative pore volume ratio determined by the formula (1) refers to the ratio of the content by volume of the nonvolatile component to the pore volume of the porous silica particles.

In the composite silica particles of the present invention, the relative pore volume ratio determined by the formula (1) is, from the viewpoint of suppressing a decrease in the amount of ceramides released into oleic acid, a major component of sebum, 110% or less, preferably 100% or less, more preferably 80% or less, further preferably 70% or less, furthermore preferably 60% or less, and, from the viewpoint of increasing the amount of supported ceramides and further increasing the amount of ceramides released into oleic acid, a major component of sebum, preferably 5% or more, more preferably 10% or more, further preferably 25% or more, furthermore preferably 35% or more. From these viewpoints, it is preferably 5% or more and 110% or less, more preferably 10% or more and 100% or less, further preferably 25% or more and 80% or less, furthermore preferably 35% or more and 70% or less, furthermore preferably 35% or more and 60% or less.

### <Porous silica particles>

In the present invention, from the viewpoint of supporting the nonvolatile component containing ceramides in the pores, the porous silica particles have a pore volume of 0.5 mL/g or more.

The pore volume of the porous silica particles is, from the viewpoint of maintaining ceramides in an amorphous state in the pores, 0.5 mL/g or more, preferably 1.0 mL/g or more, more preferably 1.2 mL/g or more, further preferably 1.4 mL/g or more, and, from the viewpoint of ensuring the strength of the particles, preferably 8.0 mL/g or less, more preferably 5.0 mL/g or less, further preferably 3.0 mL/g or less, furthermore preferably 2.0 mL/g or less. From these viewpoints, it is preferably 0.5 mL/g or more and 8.0 mL/g or less, more preferably 1.0 mL/g or more and 5.0 mL/g or less, further preferably 1.2 mL/g or more and 3.0 mL/g or less, furthermore preferably 1.4 mL/g or more and 2.0 mL/g or less.

The pore volume of the porous silica particles (hereinafter also referred to as "BJH pore volume") is a value obtained by drying a sample to be measured under vacuum at 1 kPa or less at a temperature of 200°C for 3 hours or more, then obtaining only the adsorption branch of a nitrogen adsorption isotherm at liquid-nitrogen temperature, and analyzing the adsorption isotherm by the BJH method (Barrett, E.P.; Joyner, L.G.; Halenda, P.P., J. Am. Chem. Soc., 73, 373 (1951)). In the present invention, the pore volume is defined as the cumulative pore volume of pores having radii in the range of 1 to 100 nm as measured in the above manner.

The porous silica particles have a specific surface area as determined by the BET method (BET specific surface area) of, from the viewpoint of supporting the nonvolatile component and maintaining ceramides in an amorphous state, preferably 200 m2/g or more, more preferably 300 m2/g or more, further preferably 500 m2/g or more, and, from the viewpoint of ensuring the strength of the particles, preferably 1,500 m2/g or less, more preferably 1,000 m2/g or less, further preferably 850 m2/g or less. From these viewpoints, it is preferably 200 m2/g or more and 1,500 m2/g or less, more preferably 300 m2/g or more and 1,000 m2/g or less, further preferably 500 m2/g or more and 850 m2/g or less.

The specific surface area as determined by the BET method is a value obtained by drying a sample to be measured under vacuum at 1 kPa or less at a temperature of 200°C for 3 hours or more, then obtaining only the adsorption branch of a nitrogen adsorption isotherm at liquid-nitrogen temperature, and analyzing the adsorption isotherm by the BET method.

The porous silica particles have an average particle size of, from the viewpoint of feel when used in cosmetic preparations and the like, preferably 1.0 µm or more, more preferably 2.0 µm or more, further preferably 3.0 µm or more, furthermore preferably 5.0 µm or more, and, from the same viewpoint, i.e., the viewpoint of feel when used in cosmetic preparations and the like, preferably 50.0 µm or less, more preferably 30.0 µm or less, further preferably 20.0 µm or less, furthermore preferably 15.0 µm or less. From this viewpoint, it is preferably 1.0 µm or more and 50.0 µm or less, more preferably 2.0 µm or more and 30.0 µm or less, further preferably 3.0 µm or more and 20.0 µm or less, furthermore preferably 5.0 µm or more and 15.0 µm or less.

The average particle size of the porous silica particles refers to the median diameter D₅₀ (hereinafter also simply referred to as "median diameter") in the volume-based particle size distribution as measured by laser diffraction, and is specifically measured by the method described in the Examples.

The porous silica particles have an oil absorption of, from the viewpoint of supporting the nonvolatile component, preferably 100 mL/100 g or more, more preferably 150 mL/100 g or more, further preferably 200 mL/100 g or more, and, from the viewpoint of the strength of the particles, preferably 800 mL/100 g or less, more preferably 700 mL/100 g or less, further preferably 600 mL/100 g or less.

It should be noted that the oil absorption of the porous silica particles is a value measured by the method described in "Refined Linseed Oil Method" in JIS K 5101-13-1. The oil absorption measured by the above method also includes the amount of oil retained between the particles.

The shape of the porous silica particles is not particularly limited; however, from the viewpoint of feel when used in cosmetic preparations and the like, the particles are preferably spherical.

The shape of the porous silica particles is not particularly limited, and the particles may be irregular in shape, spherical, oval, polyhedral, prismatic, and the like. The particles are usually spherical or oval. Further, particles having two or more different shapes may be used. The shape of the porous silica particles is not particularly limited; however, from the viewpoint of feel when used in cosmetic preparations and the like, the particles are preferably spherical.

### <Nonvolatile component>

The composite silica particles of the present invention contain a nonvolatile component containing ceramides. In the present invention, "nonvolatile component" refers to a compound having a boiling point (at normal pressure of 1,013 hPa) of 150°C or higher. From the viewpoint of suppressing volatilization from the composite silica particles, the boiling point of the nonvolatile component is 150°C or higher, preferably 180°C or higher, further preferably 200°C or higher. The upper limit of the boiling point of the nonvolatile component is not particularly limited; however, it is preferably 1,000°C or lower.

The nonvolatile component has a molecular weight of, from the viewpoint of increasing the boiling point, preferably 80 or more, more preferably 100 or more, and, from the viewpoint of supporting the nonvolatile component in the pores of the porous silica particles, preferably 6,000 or less, more preferably 3,000 or less, further preferably 2,000 or less, furthermore preferably 1,500 or less, furthermore preferably 1,000 or less. From these viewpoints, it is preferably 80 or more and 6,000 or less, more preferably 100 or more and 3,000 or less, further preferably 100 or more and 2,000 or less, furthermore preferably 100 or more and 1,500 or less, furthermore preferably 100 or more and 1,000 or less.

### (Ceramides)

In the present invention, as ceramides, one or more selected from natural ceramides and pseudo-ceramides can preferably be used.

Preferable examples of natural ceramides include glycoceramide, ceramide EOS, ceramide EOP, ceramide NS, ceramide NG, ceramide NP, ceramide EOH, ceramide AS, ceramide AG, and ceramide AP.

Examples of commercially available products of natural ceramides include Ceramide I, Ceramide III, Ceramide IIIB, and Ceramide VI (available from Evonik), Ceramide TIC-001 (available from TAKASAGO INTERNATIONAL CORPORATION), CERAMIDE II (available from CRODA), DS-Ceramide VI, C6-Phytoceramide, and DS-ceramide Y3S (available from DOOSAN), and CERAMIDE 2 (available from CRODA).

The pseudo-ceramide is preferably one represented by the following general formula (1):

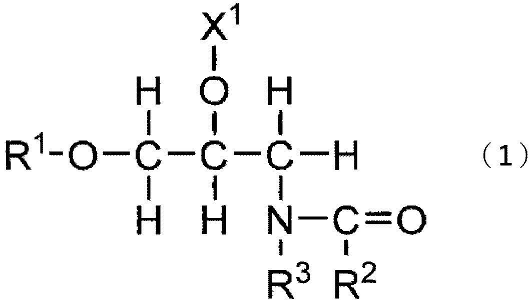

In the general formula (1), R¹ represents a C10 to C22 linear, branched, or cyclic, saturated or unsaturated hydrocarbon group optionally substituted with a hydroxyl group, or a hydrogen atom; X¹ represents a hydrogen atom, an acetyl group, or a glyceryl group; R² represents a C5 to C22 linear, branched, or cyclic, saturated or unsaturated hydrocarbon group optionally substituted with a hydroxyl group or an amino group, or a group in which a C8 to C22 linear or branched, saturated or unsaturated fatty acid optionally substituted with a hydroxyl group is ester-bonded to the w-end of the hydrocarbon group; and R³ represents a hydrogen atom or an alkyl group having a total of 1 to 30 carbons and optionally substituted with a hydroxyl group, a hydroxyalkoxy group, an alkoxy group, or an acetoxy group.

R2 is preferably a nonyl group, a tridecyl group, a pentadecyl group, an undecyl group in which linoleic acid is ester-bonded at the ω-position, a pentadecyl group in which linoleic acid is ester-bonded at the ω-position, a pentadecyl group in which 12-hydroxystearic acid is ester-bonded at the ω-position, or an undecyl group in which a methyl-branched isostearic acid is amide-bonded at the ω-position.

When R1 is a hydrogen atom, R3 is an alkyl group having a total of 10 to 30 carbons, preferably having a total of 12 to 20 carbons, and optionally substituted with a hydroxyl group, a hydroxyalkoxy group, an alkoxy group, or an acetoxy group. When R1 is a C10 to C22 linear, branched, or cyclic, saturated or unsaturated hydrocarbon group optionally substituted with a hydroxyl group, R3 preferably represents a hydrogen atom or an alkyl group having a total of 1 to 8 carbons and optionally substituted with a hydroxyl group, a hydroxyalkoxy group, an alkoxy group, or an acetoxy group. The hydroxyalkoxy group or alkoxy group of R3 preferably has 1 to 7 carbons.

The pseudo-ceramide represented by the general formula (1) is preferably one or more selected from N-(hexadecyloxyhydroxypropyl)-N-hydroxyethylhexadecanamide, N-(2-hydroxy-3-hexadecyloxypropyl)-N-2-hydroxyethylhexadecanamide, N-(2-hydroxy-3-hexadecyloxypropyl)-N-2-hydroxyethyldecanamide, and N-(tetradecyloxyhydroxypropyl)-N-hydroxyethyldecanamide, more preferably N-(hexadecyloxyhydroxypropyl)-N-hydroxyethyldecanamide.

In the present invention, since the ceramides are supported in the pores of the porous silica particles, they are present in an amorphous state in the composite silica particles. That is, in the composite silica particles of the present invention, the ceramides are preferably in an amorphous state.

Since ceramides in an amorphous state dissolve more readily in oleic acid and the like than in a crystalline state, the composite silica particles of the present invention can readily release the ceramides upon contact with oleic acid, a major component of sebum.

It should be noted that, in the present invention, the crystalline state of the ceramides in the composite silica particles is judged on the basis of the presence or absence of an endothermic peak of 1.0 J/g or more as measured using a differential scanning calorimeter under the conditions of a temperature range of 25 to 90°C and a heating rate of 1°C/min.

When an endothermic peak of 1.0 J/g or more is present, the ceramides are judged to be crystalline. On the other hand, when an endothermic peak of 1.0 J/g or more is not present, the ceramides are judged not to be crystalline, that is, are judged to be in an amorphous state. The crystalline state of the ceramides is specifically judged by the method described in the Examples.

In the present invention, the content of the ceramides in the nonvolatile component is, from the viewpoint of increasing the amount of supported ceramides and further increasing the amount of ceramides released into oleic acid, a major component of sebum, preferably 5 mass% or more, more preferably 10 mass% or more, further preferably 12 mass% or more, furthermore preferably 15 mass% or more, and, from the viewpoint of suppressing crystallization of ceramides due to an excessive amount of supported ceramides, preferably 100 mass% or less, more preferably 80 mass% or less, further preferably 50 mass% or less, furthermore preferably 30 mass% or less. From these viewpoints, it is preferably 5 mass% or more and 100 mass% or less, more preferably 10 mass% or more and 80 mass% or less, further preferably 12 mass% or more and 50 mass% or less, furthermore preferably 15 mass% or more and 30 mass% or less.

In the present invention, the content of the ceramides in the composite silica particles is, from the viewpoint of increasing the amount of supported ceramides and further increasing the amount of ceramides released into oleic acid, a major component of sebum, preferably 3 mass% or more, more preferably 4 mass% or more, further preferably 5 mass% or more, furthermore preferably 6 mass% or more, and, from the viewpoint of suppressing crystallization of ceramides due to an excessive amount of supported ceramides, preferably 80 mass% or less, more preferably 50 mass% or less, further preferably 30 mass% or less, furthermore preferably 10 mass% or less. From these viewpoints, it is preferably 3 mass% or more and 80 mass% or less, more preferably 4 mass% or more and 50 mass% or less, further preferably 5 mass% or more and 30 mass% or less, furthermore preferably 6 mass% or more and 10 mass% or less.

### (Alcohol)

In the composite silica particles of the present invention, the nonvolatile component preferably contains an alcohol. The alcohol preferably has a solubility in 100 g of water (at 25°C, and normal pressure of 1,013 hPa) of more than 1 g. The solubility may be measured by referring, for example, to Journal of the Chemical Society of Japan, 1985, No. 11, pp. 2,116-2,119, Journal of the Chemical Society of Japan, 1982, No. 11, pp. 1,830-1,834, and the like.

Since alcohols have an affinity for ceramides, the composite silica particles of the present invention containing an alcohol can further increase the amount of ceramides released into oleic acid, a major component of sebum.

The alcohol may have a substituent and a functional group.

Examples of substituents include an alkoxy group having 1 or more and 3 or less carbons and an acetyl group. Examples of functional groups include an ether group and an ester group.

In the present invention, the alcohol preferably has 2 or more hydroxy groups, more preferably has 2 or 3 hydroxy groups, further preferably has 2 hydroxy groups. That is, in the present invention, the alcohol is preferably a polyhydric alcohol having 2 or more hydroxy groups, more preferably one or more selected from dihydric alcohols and trihydric alcohols, further preferably a dihydric alcohol. When the alcohol has a substituent, one or more hydroxy groups may be substituted with an ether group or an ester group.

From the viewpoint of suppressing volatilization from the composite silica particles, the alcohol has a boiling point (at normal pressure of 1,013 hPa) of preferably 150°C or higher, more preferably 180°C or higher, further preferably 200°C or higher. The upper limit of the boiling point of the alcohol is not particularly limited; however, it is preferably 500°C or lower, more preferably 450°C or lower, further preferably 400°C or lower.

Further, from the viewpoint of an affinity for porous silica particles, the alcohol has a melting point (at normal pressure of 1,013 hPa) of preferably lower than 25°C, more preferably 20°C or lower.

The alcohol is preferably one or more selected from glycol solvents and glycerin solvents. From the viewpoint of further increasing, by virtue of an affinity for ceramides, the amount of ceramides released into oleic acid, a major component of sebum, the alcohol is more preferably a glycol solvent.

From the viewpoint of an affinity for ceramides, the glycol solvent preferably has 2 or more and 12 or less carbons, more preferably has 3 or more and 8 or less carbons, further preferably has 4 or more and 7 or less carbons.

Further, the glycol solvent may be alkoxylated with a hydrocarbon having 1 or more and 3 or less carbons, or acetylated.

Examples of glycol solvents as described above include monoalkylene glycols such as ethylene glycol (boiling point: 197°C), propylene glycol (boiling point: 188°C), 1,3-propanediol (boiling point: 211°C), 1,3-butylene glycol (1,3-butanediol) (boiling point: 203°C), and 1,2-pentanediol (boiling point: 206°C); and polyalkylene glycols such as diethylene glycol (boiling point: 245°C), dipropylene glycol (boiling point: 232°C), triethylene glycol (boiling point: 276°C), polyethylene glycol (boiling point: 250°C), and polypropylene glycol (boiling point: 287°C).

The glycol solvent may be alkoxylated with a hydrocarbon having 1 or more and 3 or less carbons, or acetylated. Examples include dipropylene glycol monomethyl ether (boiling point: 190°C), methoxyethyl carbitol acetate (boiling point: 218°C), butyl carbitol (diethylene glycol monobutyl ether) (boiling point: 230°C), methoxybutyl acetate (boiling point: 171°C), and the like.

Among these, the glycol solvent is preferably one or more selected from monoalkylene glycols and polyalkylene glycols, more preferably a polyalkylene glycol, further preferably dipropylene glycol (boiling point: 232°C).

Examples of glycerin solvents include glycerin (boiling point: 290°C), diglycerin (boiling point: 265°C), triglycerin (boiling point: 276°C), and the like.

In the present invention, the content of the alcohol in the nonvolatile component is, from the viewpoint of further increasing, by virtue of an affinity for ceramides, the amount of ceramides released into oleic acid, a major component of sebum, preferably 10 mass% or more, more preferably 20 mass% or more, further preferably 40 mass% or more, and, from the viewpoint of supporting ceramides, preferably 90 mass% or less, more preferably 85 mass% or less, further preferably 80 mass% or less. From these viewpoints, it is preferably 10 mass% or more and 90 mass% or less, more preferably 20 mass% or more and 85 mass% or less, further preferably 40 mass% or more and 80 mass% or less.

Further, in the present invention, the preferred contents of the glycol solvent and dipropylene glycol in the nonvolatile component are also in the same range as described above.

In the present invention, the mass ratio of the alcohol to the ceramides (alcohol/ceramides) is, from the viewpoint of further increasing, by virtue of an affinity for ceramides, the amount of ceramides released into oleic acid, a major component of sebum, preferably 0.1 or more, more preferably 1.0 or more, further preferably 2.0 or more, and, from the viewpoint of supporting ceramides, preferably 20.0 or less, more preferably 10.0 or less, further preferably 6.0 or less. From these viewpoints, it is preferably 0.1 or more and 20.0 or less, more preferably 1.0 or more and 10.0 or less, further preferably 2.0 or more and 6.0 or less.

Further, in the present invention, the preferred mass ratios of the glycol solvent and dipropylene glycol to the ceramides are also in the same range as described above.

In the present invention, the content of the alcohol in the composite silica particles is, from the viewpoint of further increasing, by virtue of an affinity for ceramides, the amount of ceramides released into oleic acid, a major component of sebum, preferably 5 mass% or more, more preferably 10 mass% or more, further preferably 20 mass% or more, and, from the viewpoint of supporting ceramides, preferably 40 mass% or less, more preferably 35 mass% or less, further preferably 30 mass% or less. From these viewpoints, it is preferably 5 mass% or more and 40 mass% or less, more preferably 10 mass% or more and 35 mass% or less, further preferably 20 mass% or more and 30 mass% or less.

Further, in the present invention, the preferred content of the glycol solvent in the composite silica particles is also in the same range as described above.

### (Surfactant)

In the composite silica particles of the present invention, the nonvolatile component preferably contains a surfactant. Since surfactants have an affinity for ceramides, the composite silica particles of the present invention containing a surfactant can further increase the amount of ceramides released into oleic acid, a major component of sebum.

The surfactant has a hydrocarbon group which preferably has 8 or more carbons, more preferably has 12 or more carbons, and preferably has 24 or less carbons, more preferably has 22 or less carbons.

The surfactant is preferably one or more selected from anionic surfactants, cationic surfactants, nonionic surfactants, and amphoteric surfactants, more preferably one or more selected from anionic surfactants, cationic surfactants, and nonionic surfactants, further preferably one or more selected from anionic surfactants and nonionic surfactants.

Examples of anionic surfactants include:
fatty acid salts such as sodium laurate, potassium laurate, and potassium palmitate;
polyoxyethylene alkyl ether carboxylates such as sodium polyoxyethylene tridecyl ether acetate;
alkyl phosphates such as potassium lauryl phosphate, sodium lauryl phosphate, arginine lauryl phosphate, potassium myristyl phosphate, sodium myristyl phosphate, arginine myristyl phosphate, potassium palmityl phosphate, sodium palmityl phosphate, and arginine palmityl phosphate;
polyoxyethylene alkyl ether phosphates such as sodium polyoxyethylene oleyl ether phosphate and sodium polyoxyethylene stearyl ether phosphate;
alkyl sulfates such as sodium lauryl sulfate and potassium lauryl sulfate;
polyoxyethylene alkyl ether sulfates such as potassium polyoxyethylene lauryl sulfate, sodium polyoxyethylene lauryl sulfate, and triethanolamine polyoxyethylene lauryl sulfate;
acylated amino acid salts such as sodium lauroyl sarcosinate, monosodium N-lauroyl glutamate, disodium N-stearoyl glutamate, monosodium N-myristoyl-L-glutamate, triethanolamine N-lauroyl glycinate, potassium N-coconut oil fatty acid acyl glycinate, triethanolamine N-lauroyl-β-alaninate, and triethanolamine N-stearoyl-β-alaninate;
fatty acid amide sulfonates such as sodium N-myristoyl-N-methyl taurate and sodium N-stearoyl-N-methyl taurate;
sulfosuccinates such as sodium di-2-ethylhexyl sulfosuccinate; and the like.

Among these, the anionic surfactant is preferably a fatty acid amide sulfonate, more preferably sodium N-stearoyl-N-methyl taurate.

Examples of cationic surfactants include primary amine compounds, secondary amine compounds, tertiary amine compounds, and quaternary ammonium salts. As the primary amine compound, the secondary amine compound, or the tertiary amine compound, an organic acid salt and/or an inorganic acid salt thereof can be used. Examples of primary amine compounds include long-chain alkyl amines such as lauryl amine, myristyl amine, and palmityl amine, and the like. Examples of secondary amine compounds include dialkyl amines having a long-chain alkyl group and a short-chain alkyl group, such as long-chain alkyl methyl amines, long-chain alkyl ethyl amines, and long-chain alkyl propyl amines, sphingosines such as 4D-hydroxysphinganine and 1-(2-hydroxyethylamino)-3-isostearyloxy-2-propanol, and the like. Examples of tertiary amine compounds include trialkylamines having a long-chain alkyl group and short-chain alkyl groups, such as long-chain alkyl dimethylamines, long-chain alkyl diethylamines, and long-chain alkyl dipropylamines; long-chain fatty acid dialkylaminoethylamides and long-chain fatty acid dialkylaminopropylamides, such as stearic acid dimethylaminopropylamide and stearic acid diethylaminoethylamide; alkyloxypropyl dimethylamines such as hexadecyloxypropyl dimethylamine, stearoxypropyl dimethylamine, stearoxyethyl dimethylamine, octadecyloxypropyl dimethylamine (N,N-dimethyl-3-octadecyloxypropylamine), and behenyloxypropyl dimethylamine; acyloxyethyl dialkylamines and acyloxypropyl dialkylamines; and the like. Examples of quaternary ammonium salts include alkyl trimethyl ammonium salts such as octyltrimethylammonium, decyltrimethylammonium chloride, lauryltrimethylammonium chloride, and tetradecyltrimethylammonium chloride; dialkyldimethylammonium salts such as didecyldimethylammonium chloride and distearyldimethylammonium chloride; and the like.

Among these, the cationic surfactant is preferably a secondary amine compound, more preferably a sphingosine, further preferably 1-(2-hydroxyethylamino)-3-isostearyloxy-2-propanol.

When the primary amine compounds, secondary amine compounds, and tertiary amine compounds are used as salts, examples of inorganic acids used to form the salts include phosphoric acid, hydrochloric acid, nitric acid, sulfuric acid, perchloric acid, carbonic acid, and the like. Further, examples of organic acids used to form the salts include monocarboxylic acids such as formic acid, acetic acid, propionic acid, butyric acid, isobutyric acid, and valeric acid; dicarboxylic acids such as succinic acid, phthalic acid, fumaric acid, oxalic acid, malonic acid, and glutaric acid; oxycarboxylic acids such as glycolic acid, citric acid, lactic acid, pyruvic acid, malic acid, and tartaric acid; amino acids such as L-glutamic acid and aspartic acid; and the like. Among these, an organic acid is preferred, an amino acid is more preferred, and L-glutamic acid is further preferred.

Examples of nonionic surfactants include sorbitan fatty acid esters such as sorbitan monostearate; glycerin fatty acid esters; polyglycerin fatty acid esters such as polyglyceryl monoisostearate; propylene glycol fatty acid esters; polyoxyethylene fatty acid esters such as polyethylene glycol monolaurate; sucrose fatty acid esters; polyoxyethylene sorbitan fatty acid esters such as polyoxyethylene sorbitan monostearate, polyoxyethylene sorbitan monostearate, and polyoxyethylene coconut oil fatty acid sorbitan; polyoxyethylene alkyl ethers; polyoxyethylene sorbitol fatty acid esters; polyoxyethylene glycerin fatty acid esters; polyoxyethylene propylene glycol fatty acid esters; polyoxyethylene castor oil; polyoxyethylene hydrogenated castor oil; polyoxyethylene hydrogenated castor oil fatty acid esters; alkyl polyglucosides; polyoxyalkylene-modified silicones such as polyoxyethylene/methyl polysiloxane copolymers; and the like.

Among these, the nonionic surfactant is preferably polyoxyethylene hydrogenated castor oil.

Examples of amphoteric surfactants include betaine amphoteric surfactants such as lauryl dimethyl amino acetic acid betaine, lauroyl amide betaine, and lauryl sulfobetaine.

In the present invention, the content of the surfactant in the nonvolatile component (wherein the surfactant includes a salt; the same applies hereinafter) is, from the viewpoint of further increasing, by virtue of an affinity for ceramides, the amount of ceramides released into oleic acid, a major component of sebum, preferably 1 mass% or more, more preferably 3 mass% or more, further preferably 5 mass% or more, and, from the viewpoint of supporting ceramides, preferably 50 mass% or less, more preferably 40 mass% or less, further preferably 30 mass% or less. From these viewpoints, it is preferably 1 mass% or more and 50 mass% or less, more preferably 3 mass% or more and 40 mass% or less, further preferably 5 mass% or more and 30 mass% or less.

In the present invention, the mass ratio of the surfactant to the ceramides (surfactant/ceramides) is, from the viewpoint of further increasing, by virtue of an affinity for ceramides, the amount of ceramides released into oleic acid, a major component of sebum, preferably 0.01 or more, more preferably 0.05 or more, further preferably 0.1 or more, furthermore preferably 0.3 or more, and, from the viewpoint of supporting ceramides, preferably 10.0 or less, more preferably 5.0 or less, further preferably 3.0 or less, furthermore preferably 1.0 or less. From these viewpoints, it is preferably 0.01 or more and 10.0 or less, more preferably 0.05 or more and 5.0 or less, further preferably 0.1 or more and 3.0 or less, furthermore preferably 0.3 or more and 1.0 or less.

In the present invention, the content of the surfactant in the composite silica particles is, from the viewpoint of further increasing, by virtue of an affinity for ceramides, the amount of ceramides released into oleic acid, a major component of sebum, preferably 1 mass% or more, more preferably 1.5 mass% or more, further preferably 2 mass% or more, and, from the viewpoint of supporting ceramides, preferably 15 mass% or less, more preferably 12 mass% or less, further preferably 6 mass% or less. From these viewpoints, it is preferably 1 mass% or more and 15 mass% or less, more preferably 1.5 mass% or more and 12 mass% or less, further preferably 2 mass% or more and 6 mass% or less.

### (Oily agent)

In the composite silica particles of the present invention, the nonvolatile component preferably contains an oily agent. Since oily agents have an affinity for ceramides, the composite silica particles of the present invention containing an oily agent can further increase the amount of ceramides released into oleic acid, a major component of sebum.

From the viewpoint of an affinity for ceramides, the oily agent is preferably a compound having low solubility in water. Therefore, the oily agent has a solubility in 100 g of water (at 25°C, and normal pressure of 1,013 hPa) of preferably 1 g or less, more preferably 0.1 g or less, further preferably 0.001 g or less.

The solubility may be measured by referring, for example, to Journal of the Chemical Society of Japan, 1985, No. 11, pp. 2,116-2,119, Journal of the Chemical Society of Japan, 1982, No. 11, pp. 1,830-1,834, and the like.

Examples of oily agents include liquid oils and solid oils. The oily agent is preferably one or more selected from liquid oils and solid oils, more preferably a solid oil.

It should be noted that, in the present invention, the oily agent does not include the above-described alcohol having a solubility in 100 g of water (at 25°C, and normal pressure of 1,013 hPa) of more than 1 g.

### [Liquid oil]

Examples of liquid oils include hydrocarbon oils, ether oils, ester oils, alcohols other than the above-described alcohol having a solubility in 100 g of water (at 25°C, and normal pressure of 1,013 hPa) of more than 1 g, silicone oils, fluorinated oils, and the like, each having a melting point (at normal pressure of 1,013 hPa) of lower than 25°C.

Specific examples of liquid oils include:
hydrocarbon oils such as liquid paraffin, squalane, and petroleum jelly;
ether oils such as cetyl dimethyl butyl ether, ethylene glycol dioctyl ether, and glycerol monooleyl ether;
ester oils such as octyl dodecyl myristate, isopropyl palmitate, butyl stearate, di-2-ethylhexyl adipate, neopentyl glycol dicaprate, trioctanoin, and vegetable oils such as olive oil;
alcohols such as oleyl alcohol and 2-octyldodecan-1-ol;
silicone oils such as dimethylpolysiloxane, cyclic dimethylpolysiloxane, methylphenylpolysiloxane, amino-modified silicone, carboxy-modified silicone, alcohol-modified silicone, alkyl-modified silicone, polyether-modified silicone, and fluorine-modified silicone;
fluorine oils such as perfluoroalkyl ethyl phosphate, perfluoroalkyl polyoxyethylene phosphate, perfluoro polyether, polytetrafluoroethylene; and the like.

### [Solid oil]

The solid oil has a melting point (at normal pressure of 1,013 hPa) of 25°C or higher.

Examples of solid oils include higher alcohols including sterols (excluding the above-described alcohol); higher fatty acids; hydrocarbon oils such as petroleum jelly; and the like. Among these, from the viewpoint of further increasing, by virtue of an affinity for ceramides, the amount of ceramides released into oleic acid, a major component of sebum, the solid oil is preferably a sterol.

Examples of sterols include animal-derived sterols, plant-derived sterols, and the like. Examples of animal-derived sterols include cholesterol, dihydrocholesterol, cholesterol succinate, and the like. Examples of plant-derived sterols include sitosterol, stigmasterol, campesterol, and the like. Among these, the sterol is preferably an animal-derived sterol, more preferably cholesterol.

In the present invention, the content of the oily agent in the nonvolatile component is, from the viewpoint of further increasing, by virtue of an affinity for ceramides, the amount of ceramides released into oleic acid, a major component of sebum, preferably 0.5 mass% or more, more preferably 1 mass% or more, further preferably 3 mass% or more, furthermore preferably 5 mass% or more, and, from the viewpoint of supporting ceramides, preferably 50 mass% or less, more preferably 30 mass% or less, further preferably 20 mass% or less, furthermore preferably 10 mass% or less. From these viewpoints, it is preferably 0.5 mass% or more and 50 mass% or less, more preferably 1 mass% or more and 30 mass% or less, further preferably 3 mass% or more and 20 mass% or less, furthermore preferably 5 mass% or more and 10 mass% or less.

In the present invention, the mass ratio of the oily agent to the ceramides (oily agent/ceramides) is, from the viewpoint of further increasing, by virtue of an affinity for ceramides, the amount of ceramides released into oleic acid, a major component of sebum, preferably 0.01 or more, more preferably 0.05 or more, further preferably 0.1 or more, and, from the viewpoint of supporting ceramides, preferably 10.0 or less, more preferably 5.0 or less, further preferably 1.0 or less. From these viewpoints, it is preferably 0.01 or more and 10.0 or less, more preferably 0.05 or more and 5.0 or less, further preferably 0.1 or more and 1.0 or less.

In the present invention, the content of the oily agent in the composite silica particles is, from the viewpoint of further increasing, by virtue of an affinity for ceramides, the amount of ceramides released into oleic acid, a major component of sebum, preferably 1 mass% or more, more preferably 1.5 mass% or more, further preferably 2 mass% or more, and, from the viewpoint of supporting ceramides, preferably 15 mass% or less, more preferably 12 mass% or less, further preferably 6 mass% or less. From these viewpoints, it is preferably 1 mass% or more and 15 mass% or less, more preferably 1.5 mass% or more and 12 mass% or less, further preferably 2 mass% or more and 6 mass% or less.

In the present invention, the mass ratio of the nonvolatile component to the porous silica particles (nonvolatile component/porous silica particles) is, from the viewpoint of supporting the nonvolatile component containing ceramides, preferably 0.01 or more, more preferably 0.05 or more, further preferably 0.1 or more, furthermore preferably 0.15 or more, and, from the viewpoint of retaining the nonvolatile component containing ceramides in the pores, preferably 4 or less, more preferably 3 or less, further preferably 2 or less, furthermore preferably 1.5 or less. From these viewpoints, it is preferably 0.01 or more and 4 or less, more preferably 0.05 or more and 3 or less, further preferably 0.1 or more and 2 or less, furthermore preferably 0.15 or more and 1.5 or less.

### <Water-soluble polymer>

The composite silica particles of the present invention preferably further contain a water-soluble polymer. Since water-soluble polymers have a good affinity for porous silica particles, the surfaces of the composite silica particles of the present invention can be modified with the water-soluble polymer. Therefore, when the composite silica particles are used in cosmetic preparations and the like, the particles blend better with the skin and provide a further improved feel. Further, since the surfaces of the porous silica particles are modified with the water-soluble polymer, ceramides are more readily released from the composite silica particles, and the amount of ceramides released into oleic acid, a major component of sebum, can be further increased.

In addition, since the water-soluble polymer functions as a granulating agent when the granulated particles containing the composite silica particles described below are produced, the granulated particles can be obtained more efficiently.

From the viewpoint of an affinity for porous silica particles, the water-soluble polymer preferably has a solubility in 100 g of water (at 25°C, and normal pressure of 1,013 hPa) of 1 g or more. The solubility may be measured by referring, for example, to Journal of the Chemical Society of Japan, 1985, No. 11, pp. 2,116-2,119, Journal of the Chemical Society of Japan, 1982, No. 11, pp. 1,830-1,834, and the like.

From the viewpoint of an affinity for porous silica particles, the water-soluble polymer has a weight average molecular weight of preferably 10,000 or more, more preferably 30,000 or more, further preferably 50,000 or more, and preferably 500,000 or less, more preferably 400,000 or less, further preferably 300,000 or less.

The weight average molecular weight of the water-soluble polymer is measured by gel permeation chromatography (GPC) using liquid chromatography, and is specifically measured by the method described in the Examples.

The water-soluble polymer is preferably one or more selected from synthetic polymers and natural polymers, and more preferably a natural polymer from the viewpoint of an affinity for porous silica particles.

Examples of synthetic polymers include salts of polyacrylic acid, poly(methacrylic acid), and the like, such as sodium salts and potassium salts thereof; polyvinylpyrrolidone; and the like.

The natural polymer is preferably a water-soluble polysaccharide. Examples of water-soluble polysaccharides include saccharides such as glucose, lactose, and sucrose; and polysaccharides containing any of these saccharides as structural units and derivatives thereof. The derivatives have an alkyl group having 1 or more and 3 or less carbons, a hydroxyalkyl group, or a polyalkylene oxide group such as a polyethylene oxide group or a polypropylene oxide group.

Specific examples of water-soluble polysaccharides include locust bean gum, guar gum, tamarind gum, quince seed-derived gum, gum arabic, tragacanth gum, karaya gum, carrageenan, pectin, hydroxypropyl guar gum, methylcellulose, hydroxyethylcellulose, hydroxypropylcellulose, carboxymethylcellulose, tuberose polysaccharide, xanthan gum, gellan gum, dextran, pullulan, and mucopolysaccharides such as hyaluronic acid, chondroitin sulfate, dermatan sulfate, chitin, and chitosan. Among these, the water-soluble polysaccharide is preferably one or more selected from xanthan gum and pullulan, more preferably pullulan.

In the present invention, the content of the water-soluble polymer per 100 parts by mass of the porous silica particles is, from the viewpoints of further increasing, by virtue of an affinity for porous silica particles, the amount of ceramides released into oleic acid, a major component of sebum, and obtaining granulated particles, preferably 0.5 parts by mass or more, more preferably 1 part by mass or more, further preferably 3 parts by mass or more, furthermore preferably 5 parts by mass or more, and, from the same viewpoints, preferably 50 parts by mass or less, more preferably 30 parts by mass or less, further preferably 20 parts by mass or less, furthermore preferably 15 parts by mass or less. From these viewpoints, it is preferably 0.5 parts by mass or more and 50 parts by mass or less, more preferably 1 part by mass or more and 30 parts by mass or less, further preferably 3 parts by mass or more and 20 parts by mass or less, furthermore preferably 5 parts by mass or more and 15 parts by mass or less. The preferred content of the water-soluble polysaccharide per 100 parts by mass of the porous silica particles is also in the same range as described above.

In the present invention, the content of the water-soluble polymer in the composite silica particles is, from the viewpoints of further increasing, by virtue of an affinity for porous silica particles, the amount of ceramides released into oleic acid, a major component of sebum, and obtaining granulated particles, preferably 1 mass% or more, more preferably 2 mass% or more, further preferably 3 mass% or more, and, from the viewpoint of supporting ceramides, preferably 18 mass% or less, more preferably 14 mass% or less, further preferably 8 mass% or less.

In the present invention, from the viewpoint of supporting the nonvolatile component containing ceramides in the pores, the composite silica particles have a moisture content (water content) of preferably 10 mass% or less, more preferably 7 mass% or less, further preferably 5 mass% or less, and preferably 0.01 mass% or more.

In the present invention, the composite silica particles have an average particle size of, from the viewpoint of supporting the nonvolatile component, preferably 1.0 µm or more, more preferably 3.0 µm or more, further preferably 5.0 µm or more, and, from the viewpoint of feel when used in cosmetic preparations and the like, preferably 50.0 µm or less, more preferably 30.0 µm or less, further preferably 20.0 µm or less, furthermore preferably 15.0 µm or less.

The average particle size of the composite silica particles refers to the median diameter D₅₀ (hereinafter also simply referred to as "median diameter") in the volume-based particle size distribution as measured by laser diffraction, and is specifically measured by the method described in the Examples.

Further, the composite silica particles of the present invention are preferably in powder form. The powder form can be confirmed by observing, with an optical microscope, that the particles are present separately from one another.

### <Method for producing the composite silica particles>

A method for producing the composite silica particles of the present invention comprises the following steps 1 and 2 in this order:
step 1: a step of mixing an emulsion or a solution containing the nonvolatile component containing ceramides with the porous silica particles to obtain a suspension; and
step 2: a step of drying the suspension obtained in step 1.

### (Step 1)

Step 1 is a step of mixing an emulsion or a solution containing the nonvolatile component containing ceramides with the porous silica particles to obtain a suspension.

### [Emulsion]

The emulsion containing the nonvolatile component containing ceramides can be obtained by mixing an oil phase containing ceramides and a water phase containing a surfactant.

That is, when the emulsion is used in step 1, the method preferably comprises the following step A before step 1:
step A: a step of mixing an oil phase containing ceramides and a water phase containing a surfactant to obtain an emulsion containing the nonvolatile component containing ceramides.

A solvent used in the oil phase is preferably an organic solvent having a boiling point (at normal pressure of 1,013 hPa) of 100°C or lower, more preferably one or more selected from ethanol, acetone, and methyl ethyl ketone, further preferably ethanol. Since the solvent used in the oil phase has a boiling point of 100°C or lower, the solvent can be removed from the resulting composite silica particles by drying the suspension in step 2.

In the method for producing the composite silica particles of the present invention, when the above-described alcohol, surfactant, and oily agent are used in the nonvolatile component in the composite silica particles, these components are preferably contained in the oil phase.

A solvent used in the water phase is preferably water, such as deionized water.

In the method for producing the composite silica particles of the present invention, from the viewpoint of efficiently obtaining the emulsion, the water phase preferably contains a surfactant, more preferably contains an anionic surfactant.

In step 1, the content of the ceramides in the emulsion is preferably 0.1 mass% or more, more preferably 0.5 mass% or more, further preferably 1 mass% or more, and preferably 10 mass% or less, more preferably 5 mass% or less, further preferably 3 mass% or less.

In step 1, the content of the oily agent in the emulsion is preferably 0.1 mass% or more, more preferably 0.2 mass% or more, further preferably 0.3 mass% or more, and preferably 5 mass% or less, more preferably 3 mass% or less, further preferably 1 mass% or less.

In step 1, from the viewpoint of emulsification, the content of the surfactant in the emulsion is preferably 0.1 mass% or more, more preferably 0.5 mass% or more, further preferably 1 mass% or more, and preferably 10 mass% or less, more preferably 5 mass% or less, further preferably 3 mass% or less.

### [Solution]

The solution containing the nonvolatile component containing ceramides can be obtained as the oil phase by mixing a solvent and the nonvolatile component containing ceramides.

The solvent used in the solution is preferably an organic solvent having a boiling point (at normal pressure of 1,013 hPa) of 100°C or lower, more preferably one or more selected from ethanol, acetone, and methyl ethyl ketone, further preferably ethanol. Since the solvent used in the oil phase has a boiling point of 100°C or lower, the solvent can be removed from the resulting composite silica particles by drying the suspension in step 2.

In step 1, the emulsion or solution containing the nonvolatile component containing ceramides may be mixed with the porous silica particles by a well-known method, and examples include mixing using an impeller, and the like.

In step 1, the mass ratio of the emulsion or solution to the porous silica particles during mixing (emulsion or solution/porous silica particles) is, from the viewpoint of supporting ceramides, preferably 0.1 or more, more preferably 0.3 or more, further preferably 0.5 or more, and, from the viewpoint of reducing workload of the drying process in step 2, preferably 50 or less, more preferably 30 or less, further preferably 20 or less.

In the method for producing the composite silica particles of the present invention, when the resulting composite silica particles contain a water-soluble polymer, the water-soluble polymer is preferably also mixed when the emulsion or solution is mixed with the porous silica particles in step 1.

That is, in the method for producing the composite silica particles of the present invention, step 1 is preferably a step of mixing an emulsion or a solution containing the nonvolatile component containing ceramides, the porous silica particles, and the water-soluble polymer to obtain a suspension.

In step 1, the content of the ceramides in the emulsion or solution is preferably 5 mass% or more, more preferably 10 mass% or more, further preferably 15 mass% or more, and preferably 35 mass% or less, more preferably 30 mass% or less, further preferably 25 mass% or less.

### (Step 2)

Step 2 is a step of drying the suspension obtained in step 1. The composite silica particles of the present invention can be obtained by drying the suspension in step 2.

As a method for drying the suspension in step 2, a well-known method may be used, and shelf drying, spray drying, or the like may be used.

When drying in step 2 is performed by shelf drying, the drying temperature is preferably 70 to 250°C, more preferably 75 to 220°C, further preferably 80 to 200°C. Further, the drying time is preferably 8 to 72 hours, more preferably 10 to 24 hours, further preferably 12 to 18 hours.

A commercially available electric dryer or the like may be used for the shelf drying.

When drying in step 2 is performed by spray drying, examples of spray drying methods include well-known methods such as a rotary atomizer method, a pressure nozzle method, a two-fluid nozzle method, and a four-fluid nozzle method. Among these, spray drying in step 2 is preferably performed by a two-fluid nozzle method.

The hot air inlet temperature in the spray drying is preferably 80 to 250°C, more preferably 100 to 220°C, further preferably 120 to 200°C. The hot air outlet temperature in the spray drying is preferably 50 to 120°C, more preferably 60 to 110°C, further preferably 70 to 100°C. The outlet temperature can be adjusted by controlling the inlet temperature.

A commercially available spray dryer may be used for the spray drying.

### [Granulated particles containing the composite silica particles]

The granulated particles of the present invention are granulated particles containing the composite silica particles. The granulated particles of the present invention contain the composite silica particles granulated with a water-soluble polymer. That is, the granulated particles of the present invention contain the composite silica particles, and preferably further contain a water-soluble polymer. As the water-soluble polymers, the same water-soluble polymers as those described above for the composite silica particles are preferably used. From the viewpoint of granulability, the water-soluble polymer is more preferably a water-soluble polysaccharide. As the granulation method, the method for producing the granulated particles containing the composite silica particles described below may be used.

Since the granulated particles of the present invention contain the composite silica particles, the granulated particles can readily release ceramides upon contact with oleic acid, a major component of sebum.

In the granulated particles of the present invention, the content of the water-soluble polymer per 100 parts by mass of the porous silica particles is, from the viewpoint of further increasing, by virtue of an affinity for porous silica particles, the amount of ceramides released into oleic acid, a major component of sebum, preferably 0.5 parts by mass or more, more preferably 1 part by mass or more, further preferably 3 parts by mass or more, furthermore preferably 5 parts by mass or more, and, from the same viewpoint, preferably 50 parts by mass or less, more preferably 30 parts by mass or less, further preferably 20 parts by mass or less, furthermore preferably 15 parts by mass or less. From this viewpoint, it is preferably 0.5 parts by mass or more and 50 parts by mass or less, more preferably 1 part by mass or more and 30 parts by mass or less, further preferably 3 parts by mass or more and 20 parts by mass or less, furthermore preferably 5 parts by mass or more and 15 parts by mass or less. The preferred content of the water-soluble polysaccharide per 100 parts by mass of the porous silica particles is also in the same range as described above.

In the present invention, from the viewpoint of supporting the nonvolatile component containing ceramides in the pores, the granulated particles have a moisture content (water content) of preferably 10 mass% or less, more preferably 7 mass% or less, further preferably 5 mass% or less, and preferably 0.01 mass% or more.

### <Method for producing the granulated particles containing the composite silica particles>

A method for producing the granulated particles of the present invention is a method for producing the granulated particles containing the composite silica particles, the method comprising the following steps 1' and 2' in this order:
step 1': a step of mixing an emulsion or a solution containing the nonvolatile component containing ceramides, the porous silica particles, and the water-soluble polymer to obtain a suspension; and
step 2': a step of spray-drying the suspension obtained in step 1',
wherein a relative pore volume ratio determined by the following formula (1) is 110% or less: 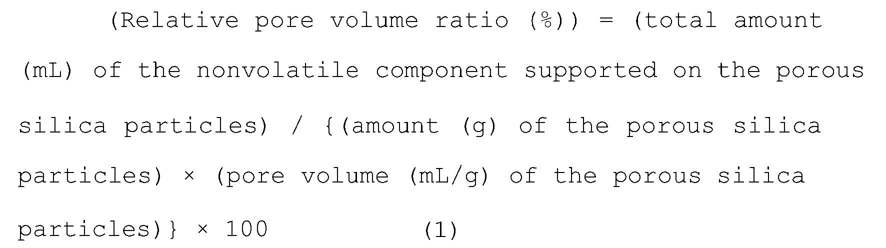 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)

### (Step 1')

Step 1' is a step of mixing an emulsion or a solution containing the nonvolatile component containing ceramides, the porous silica particles, and the water-soluble polymer to obtain a suspension.

Embodiments of the emulsion or solution containing the nonvolatile component containing ceramides in step 1' are the same as those in step 1 of the method for producing the composite silica particles.

In step 1', the same water-soluble polymers as those described above for the composite silica particles are preferably used. From the viewpoint of efficiently obtaining the granulated particles, the water-soluble polymer is more preferably a water-soluble polysaccharide. The water-soluble polysaccharides are preferably the same as those described above for the composite silica particles of the present invention.

### (Step 2')

Step 2' is a step of spray-drying the suspension obtained in step 1'.

Since the water-soluble polymer contained in the emulsion or solution in step 1' functions as a so-called granulating agent, the granulated particles of the present invention can be obtained by spray-drying the suspension in step 2'.

The spray drying in step 2' is preferably performed by any of the spray drying methods described above for step 2 of the method for producing the composite silica particles.

The present invention further discloses the following [1] to [37].
[1] Composite silica particles in which a nonvolatile component containing ceramides is supported on porous silica particles,
   wherein:
   the porous silica particles have a pore volume of 0.5 mL/g or more;
   the nonvolatile component has a boiling point (at 1,013 hPa) of 150°C or higher; and
   a relative pore volume ratio determined by the following formula (1) is 110% or less: 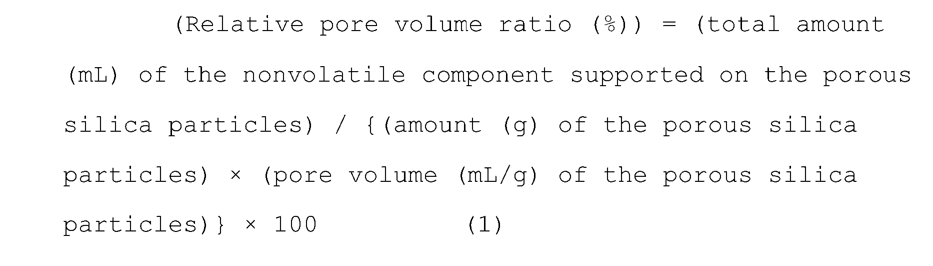 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)
[2] The composite silica particles according to [1], wherein the relative pore volume ratio determined by the formula (1) is 10% or more and 100% or less.
[3] The composite silica particles according to [1] or [2], wherein the relative pore volume ratio determined by the formula (1) is 25% or more and 80% or less.
[4] The composite silica particles according to any one of [1] to [3], wherein the porous silica particles have a pore volume of 1.0 mL/g or more and 5.0 mL/g or less.
[5] The composite silica particles according to any one of [1] to [4], wherein the porous silica particles have a pore volume of 1.2 mL/g or more and 3.0 mL/g or less.
[6] The composite silica particles according to any one of [1] to [5], wherein the ceramides are in an amorphous state.
[7] The composite silica particles according to any one of [1] to [6], wherein the content of the ceramides in the nonvolatile component is 5 mass% or more and 100 mass% or less.
[8] The composite silica particles according to any one of [1] to [7], wherein the content of the ceramides in the nonvolatile component is 10 mass% or more and 80 mass% or less.
[9] The composite silica particles according to any one of [1] to [8], wherein the content of the ceramides in the composite silica particles is 3 mass% or more and 80 mass% or less.
[10] The composite silica particles according to any one of [1] to [9], wherein the content of the ceramides in the composite silica particles is 4 mass% or more and 50 mass% or less.
[11] The composite silica particles according to claim 1 or 2, wherein the mass ratio of the nonvolatile component to the porous silica particles (nonvolatile component/porous silica particles) is 4 or less.
[12] The composite silica particles according to any one of [1] to [11], wherein the mass ratio of the nonvolatile component to the porous silica particles (nonvolatile component/porous silica particles) is 0.01 or more and 4 or less.
[13] The composite silica particles according to any one of [1] to [12], wherein the mass ratio of the nonvolatile component to the porous silica particles (nonvolatile component/porous silica particles) is 0.05 or more and 3 or less.
[14] The composite silica particles according to any one of [1] to [13], wherein:
   the nonvolatile component contains an alcohol; and
   the alcohol has a solubility in 100 g of water (at 25°C and 1,013 hPa) of more than 1 g.
[15] The composite silica particles according to [14], wherein the mass ratio of the alcohol to the ceramides (alcohol/ceramides) is 0.1 or more and 20.0 or less.
[16] The composite silica particles according to [14] or [15], wherein the mass ratio of the alcohol to the ceramides (alcohol/ceramides) is 1.0 or more and 10.0 or less.
[17] The composite silica particles according to any one of [14] to [16], wherein the alcohol contains a glycol solvent.
[18] The composite silica particles according to any one of [14] to [17], wherein the alcohol contains dipropylene glycol.
[19] The composite silica particles according to any one of [1] to [17], wherein the nonvolatile component contains a surfactant.
[20] The composite silica particles according to [19], wherein the mass ratio of the surfactant to the ceramides (surfactant/ceramides) is 0.01 or more and 10.0 or less.
[21] The composite silica particles according to [19] or [20], wherein the mass ratio of the surfactant to the ceramides (surfactant/ceramides) is 0.05 or more and 5.0 or less.
[22] The composite silica particles according to any one of [1] to [21], wherein the content of the ceramides in the nonvolatile component is 5 mass% or more.
[23] The composite silica particles according to any one of [1] to [22], further containing a water-soluble polymer.
[24] The composite silica particles according to [23], wherein the water-soluble polymer is a water-soluble polysaccharide.
[25] The composite silica particles according to [23] or [24], wherein the content of the water-soluble polymer per 100 parts by mass of the porous silica particles is 0.5 parts by mass or more and 50 parts by mass or less.
[26] The composite silica particles according to any one of [21] to [23], wherein the content of the water-soluble polymer per 100 parts by mass of the porous silica particles is 1 part by mass or more and 30 parts by mass or less.
[27] The composite silica particles according to any one of [1] to [26], wherein the composite silica particles have a moisture content of 10 mass% or less.
[28] The composite silica particles according to any one of [1] to [27], wherein the composite silica particles have a moisture content of 7 mass% or less.
[29] The composite silica particles according to any one of [1] to [28], wherein the composite silica particles are in powder form.
[30] Granulated particles containing the composite silica particles according to any one of [1] to [29].
[31] The granulated particles according to [30], further containing a water-soluble polymer.
[32] The granulated particles according to [30] or [31], wherein the granulated particles have a moisture content of 10 mass% or less.
[33] The granulated particles according to any one of [30] to [32], wherein the granulated particles have a moisture content of 7 mass% or less.
[34] The granulated particles according to any one of [30] to [33], wherein the granulated particles have a moisture content of 5 mass% or less.
[35] A method for producing the composite silica particles according to any one of [1] to [29], comprising the following steps 1 and 2 in this order:
   step 1: a step of mixing an emulsion or a solution containing the nonvolatile component containing ceramides with the porous silica particles to obtain a suspension; and
   step 2: a step of drying the suspension obtained in step 1.
[36] A method for producing the granulated particles containing the composite silica particles according to any one of [30] to [34], comprising the following steps 1' and 2' in this order:
   step 1': a step of mixing an emulsion or a solution containing the nonvolatile component containing ceramides, the porous silica particles, and a water-soluble polymer to obtain a suspension; and
   step 2': a step of spray-drying the suspension obtained in step 1'.
[37] The method for producing the granulated particles containing the composite silica particles according to [36], wherein the water-soluble polymer is a water-soluble polysaccharide.

### Examples

The present invention is specifically described below with reference to Examples; however, the present invention is not limited to these Examples. Various properties were measured and evaluated by the methods described below.

### <Production of composite silica particles>

### Examples 1 to 4 and Comparative Examples 2 to 5

### (Preparation of oil phase)

Components were mixed in accordance with the formulations shown in Table 1 to obtain the oil phases (solutions) used in Examples 1 to 4 and Comparative Examples 2 to 5. The mixing was performed using a propeller blade (diameter: 30 mm, width: 5 mm) under the conditions of a propeller blade rotation speed of 120 rpm, a mixing temperature of 60°C, and a mixing time of 10 minutes.

### (Preparation of suspension)

The oil phases (solutions) prepared in accordance with the formulations shown in Table 1 were mixed with porous silica particles or non-porous silica particles in accordance with the formulations shown in Table 1 to obtain suspensions. The mixing to obtain the suspensions was performed using a stirring bar under the conditions of a mixing temperature of 25°C and a mixing time of 5 minutes.

### (Production of composite silica particles)

10 g of each suspension was placed in a crystallizing dish and shelf-dried using an electric dryer under the conditions of a drying temperature of 90°C and a drying time of 15 hours, and then cooled at room temperature (25°C). By the shelf drying, ethanol, the solvent in the oil phase, was completely removed, and ceramides were supported on the porous silica particles or non-porous silica particles to obtain composite silica particles. The compositions, physical properties, and evaluation results of the resulting composite silica particles are shown in Table 2.

### Examples 5 to 8

### (Preparation of oil phase)

In Example 5, ceramides were melted at 80°C and used as the oil phase. Components were mixed in accordance with the formulations shown in Table 1 to obtain the oil phases used in Examples 6 to 8. The mixing was performed using a propeller blade (diameter: 30 mm, width: 5 mm) at a propeller blade rotation speed of 120 rpm and a mixing temperature of 80°C for a mixing time of 60 minutes.

### (Preparation of water phase)

Components were mixed in accordance with the formulations shown in Table 1 to obtain the water phases used in Examples 5 to 8. The mixing was performed using a propeller blade (diameter: 30 mm, width: 5 mm) under the conditions of a propeller blade rotation speed of 120 rpm, a mixing temperature of 80°C, and a mixing time of 10 minutes.

### (Preparation of emulsion)

The water phases prepared in accordance with the formulations shown in Table 1 were mixed with the oil phases prepared in accordance with the formulations shown in Table 1 to obtain emulsions.

The mixing to obtain the emulsions was performed using a propeller blade (diameter: 30 mm, width: 5 mm) under the conditions of a propeller blade rotation speed of 120 rpm, a mixing temperature of 25°C, and a mixing time of 10 minutes.

The emulsions were mixed with porous silica particles (SUNSPHERE H-52, manufactured by AGC Si-Tech Co., Ltd., pore volume: 1.5 mL/g, BET specific surface area: 700 m2/g, oil absorption: 300 mL/100 g, average particle size: 5 µm) in accordance with the formulations shown in Table 1 to obtain suspensions. The mixing to obtain the suspensions was performed using a propeller blade (diameter: 30 mm, width: 5 mm) under the conditions of a propeller blade rotation speed of 200 rpm, a mixing temperature of 25°C, and a mixing time of 60 minutes.

### (Production of composite silica particles)

10 g of each suspension was placed in a crystallizing dish and shelf-dried using an electric dryer under the conditions of a drying temperature of 90°C and a drying time of 15 hours, and then cooled at room temperature. By the shelf drying, ethanol, the solvent in the oil phase, and deionized water, the solvent in the water phase, were completely removed, and ceramides were supported on the porous silica particles to obtain composite silica particles. The compositions, physical properties, and evaluation results of the resulting composite silica particles are shown in Table 2.

### Example 9

### (Preparation of oil phase)

Components were mixed in accordance with the formulation shown in Table 1 to obtain the oil phase used in Example 9. The mixing was performed using a propeller blade (diameter: 30 mm, width: 5 mm) under the conditions of a propeller blade rotation speed of 120 rpm, a mixing temperature of 80°C, and a mixing time of 60 minutes.

### (Preparation of water phase)

Components were mixed in accordance with the formulation shown in Table 1 to obtain the water phase used in Example 9. The mixing was performed using a propeller blade (diameter: 30 mm, width: 5 mm) under the conditions of a propeller blade rotation speed of 120 rpm, a mixing temperature of 80°C, and a mixing time of 10 minutes.

### (Preparation of suspension)

The water phase prepared in accordance with the formulation shown in Table 1 was mixed with the oil phase prepared in accordance with the formulation shown in Table 1 to obtain an emulsion. The emulsion was mixed with pullulan (cosmetic-grade pullulan, manufactured by Hayashibara Co., Ltd.) as a water-soluble polymer and porous silica particles (SUNSPHERE H-52, manufactured by AGC Si-Tech Co., Ltd., pore volume: 1.5 mL/g, BET specific surface area: 700 m2/g, oil absorption: 300 mL/100 g, average particle size: 7.7 µm) in accordance with the formulation shown in Table 1 to obtain a suspension. The mixing to obtain the suspension was performed using a propeller blade (diameter: 30 mm, width: 5 mm) under the conditions of a propeller blade rotation speed of 200 rpm, a mixing temperature of 25°C, and a mixing time of 60 minutes.

### (Production of composite silica particles)

10 g of the suspension was placed in a crystallizing dish and shelf-dried using an electric dryer under the conditions of a drying temperature of 90°C and a drying time of 15 hours, and then cooled at room temperature. By the shelf drying, ethanol, the solvent in the oil phase, and deionized water, the solvent in the water phase, were completely removed, and ceramides were supported on the porous silica particles to obtain composite silica particles. The composition, physical properties, and evaluation result of the resulting composite silica particles are shown in Table 2.

### <Production of granulated particles>

### Example 10

### (Preparation of oil phase)

Components were mixed in accordance with the formulation shown in Table 1 to obtain the oil phase used in Example 10. The mixing was performed using a propeller blade (diameter: 150 mm, width: 10 mm) under the conditions of a propeller blade rotation speed of 240 rpm, a mixing temperature of 80°C, and a mixing time of 60 minutes.

### (Preparation of water phase)

Components were mixed in accordance with the formulation shown in Table 1 to obtain the water phase used in Example 10. The mixing was performed using a three-blade pitched propeller (diameter: 320 mm, width: 60 mm) under the conditions of a propeller blade rotation speed of 120 rpm, a mixing temperature of 80°C, and a mixing time of 10 minutes.

### (Preparation of suspension)

The water phase prepared in accordance with the formulation shown in Table 1 was mixed with the oil phase separately prepared in accordance with the formulation shown in Table 1 to obtain an emulsion. The emulsion was mixed with pullulan (cosmetic-grade pullulan, manufactured by Hayashibara Co., Ltd.) as a water-soluble polymer and porous silica particles (SUNSPHERE H-52, manufactured by AGC Si-Tech Co., Ltd., pore volume: 1.5 mL/g, BET specific surface area: 700 m2/g, oil absorption: 300 mL/100 g, average particle size: 5 µm) in accordance with the formulation shown in Table 1 to obtain a suspension. The mixing to obtain the suspension was performed using a three-blade pitched propeller (diameter: 320 mm, width: 60 mm) under the conditions of a propeller blade rotation speed of 400 rpm, a mixing temperature of 25°C, and a mixing time of 60 minutes.

### (Production of granulated particles)

The suspension was spray-dried using a pilot-scale co-current spray dryer (AD-0506 N/R spray dryer, manufactured by Ashizawa Niro Atomizer Co., Ltd., tower length: 8,650 mm, straight cylinder length: 6,000 mm, tower diameter: 3,200 mm) at an inlet air temperature of 180°C. The dried material obtained by the spray drying was collected at two points, namely, the tower bottom and the cyclone bottom, and the collected materials were then mixed in the mass ratio in which they were collected to obtain granulated particles containing composite silica particles.

During spray drying, the suspension was sprayed upward using a two-fluid nozzle (CNP 500, manufactured by Atomax Co., Ltd.)

Further, ethanol, the solvent in the oil phase, and deionized water, the solvent in the water phase, were removed by the spray drying.

The composition, physical properties, and evaluation result of the resulting granulated particles are shown in Table 2.

### <Ceramide particles>

### Comparative Example 1

As the ceramide particles, a ceramide material (SOFCARECERAMIDE SL-E, manufactured by Kao Corporation, average particle size: 119 µm) was used in an as-received state.

Details of the components shown in Tables 1 and 2 are as follows.

### [Silica particles]

· Porous silica particles: SUNSPHERE H-52: manufactured by AGC Si-Tech Co., Ltd., pore volume: 1.5 mL/g, BET specific surface area: 700 m2/g, oil absorption: 300 mL/100 g, average particle size: 5 µm
· Non-porous silica particles: SUNSPHERE NP-200: manufactured by AGC Si-Tech Co., Ltd., pore volume: 0.1 mL/g, BET specific surface area: 100 m2/g, oil absorption: 40 mL/100 g, average particle size: 20 µm

### [Ceramides]

· N-(Hexadecyloxyhydroxypropyl)-N-hydroxyethylhexadecanamide: SOFCARECERAMIDE SL-E, manufactured by Kao Corporation, density: 1.00 g/mL

### [Alcohol]

· Dipropylene glycol: DPG-RF, manufactured by ADEKA CORPORATION, boiling point: 232°C, density: 1.03 g/mL

### [Surfactant]

· Anionic surfactant: sodium N-stearoyl-N-methyl taurate: NIKKOL SMT, manufactured by Nikko Chemicals Co., Ltd., density: 1.00 g/mL
· Cationic surfactant: 1-(2-hydroxyethylamino)-3-isostearyloxy-2-propanol: HE-ISP, manufactured by Kao Corporation, density: 0.91 g/mL
· Nonionic surfactant: polyoxyethylene hydrogenated castor oil: EMANON CH-60 (K), manufactured by Kao Corporation, density: 1.03 g/mL

### [Oily agent]

· Liquid oil: dimethylpolysiloxane: KF-96A-6CS, manufactured by Shin-Etsu Chemical Co., Ltd., density: 1.00 g/mL
· Solid oil: cholesterol: Cholesterol JSQI, manufactured by Nippon Fine Chemical Co., Ltd., density: 1.00 g/mL

### [Organic acid]

· L-glutamic acid: L-glutamic acid, manufactured by AJINOMOTO CO., INC.

### [Water-soluble polymer]

· Pullulan (water-soluble polysaccharide): cosmetic-grade pullulan, manufactured by Hayashibara Co., Ltd., weight-average molecular weight: 200,000, density: 1.00 g/mL

### [Solvent in oil phase]

· Ethanol: Ethanol (99.5), manufactured by FUJIFILM Wako Pure Chemical Corporation

### [Solvent in water phase]

· Deionized water

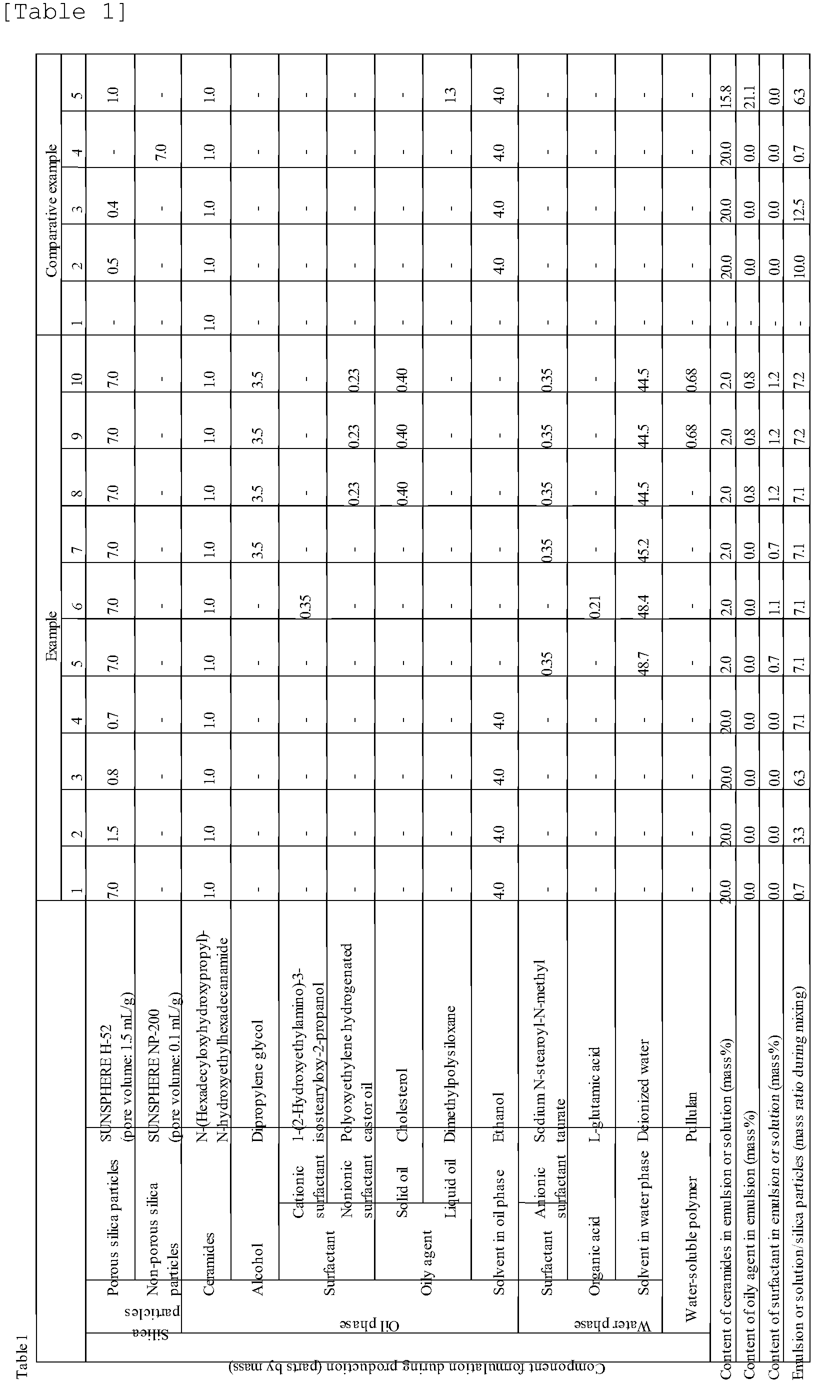

### [Evaluation and measurement method]

### <Calculation of relative pore volume ratio>

The relative pore volume ratio of the resulting composite silica particles or granulated particles was determined by the following formula (1) from the amount and pore volume of the silica particles and the amount and density of the nonvolatile component shown in Table 2: 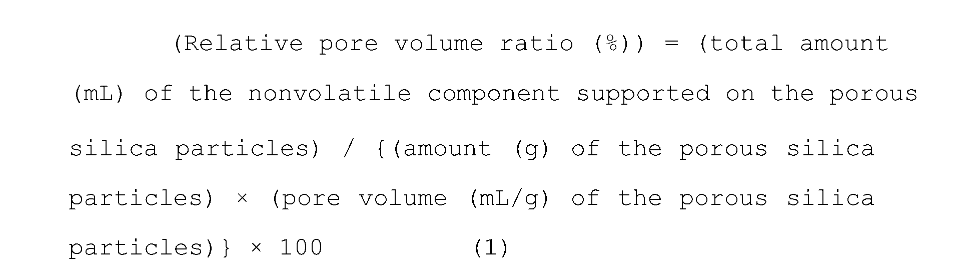 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)

### <Crystalline state of ceramides>

The composite silica particles or granulated particles of the Examples and Comparative Examples were measured using a differential scanning calorimeter (DSC600, manufactured by Hitachi High-Tech Corporation) under the following measurement conditions. The ceramides were judged to be crystalline when an endothermic peak of 1.0 J/g or more was present, and judged not to be crystalline, that is, judged to be amorphous, when no such endothermic peak was present.

### (Measurement conditions)

· Temperature range: 25 to 90°C
· Heating rate: 1°C/min.

### <Average particle size>

The average particle size of the composite silica particles or granulated particles of the Examples and Comparative Examples and that of the porous silica particles used as a raw material were measured using a laser diffraction/scattering particle size distribution analyzer LA-920 (manufactured by HORIBA, Ltd.) under the following measurement conditions. D50 (median diameter) measured by dispersing the particles in ethanol was taken as the average particle size.

### (Measurement conditions)

· Measurement temperature: 25°C
· Relative refractive index: 1.07

### <Concentration of ceramides released into oleic acid>

In a glass bottle having a capacity of 50 mL, 10 g of oleic acid (LUNAC O-V, manufactured by Kao Corporation) was placed, and the composite silica particles or granulated particles of the Examples and Comparative Examples were mixed thereinto such that the amount of ceramide was 7.2 g/L. The glass bottle was placed in a water bath at 35°C, and the mixture was stirred using a magnetic stirrer and a stirring bar (diameter: 3.0 cm) under the conditions of 300 rpm and 10 minutes to obtain a dispersion. A solid residue was then removed from the dispersion using a membrane filter having a pore size of 0.8 µm (DISMIC 25CS080AN, manufactured by ADVANTEC CO., LTD.) to obtain a liquid containing released ceramides as a measurement sample. The concentration of the ceramide material in the liquid was measured using a high-performance liquid chromatography system (Chromaster, manufactured by Hitachi High-Tech Corporation) under the following measurement conditions to determine the concentration of the ceramides released into oleic acid.

It should be noted that oleic acid is one of the major components contained in sebum, and oleic acid was used as a model of sebum in the present Examples. That is, a higher concentration of the ceramides released into oleic acid indicates a larger amount of ceramide released into sebum and thus delivered to the skin.

### (Measurement conditions)

· Detector: 5430 Diode Array Detector, manufactured by Hitachi High-Tech Corporation
· Detection method: absorbance (at 212 nm) · Column: CAPCELLPAK C18 MG, manufactured by OSAKA SODA CO., LTD.
· Solvent: a mixture of 70% by volume of acetonitrile (for high-performance liquid chromatography, manufactured by FUJIFILM Wako Pure Chemical Corporation) and 30% by volume of tetrahydrofuran (for high-performance liquid chromatography, manufactured by FUJIFILM Wako Pure Chemical Corporation)
· Measurement temperature: 40°C
· Flow rate: 1.0 mL/min.

### <Weight-average molecular weight of water-soluble polymer>

The weight-average molecular weight of the water-soluble polymer was measured by gel permeation chromatography (GPC) under the following conditions.

### <Measurement conditions>

Columns: TSKgel GMPWXL + TSKgel GMPWXL (manufactured by Tosoh Corporation)
Eluent: 0.2 M phosphate buffer/CH₃CN = 7/3 (volume ratio) Temperature: 40°C
Flow rate: 1.0 mL/min.
Sample concentration: 2 mg/mL
Detector: RI
Standard: sodium polystyrenesulfonate (weight-average
molecular weight: 1,100, 3,610, 14,900, 152,000, manufactured by Polymer Standards Service)

### <Moisture content>

The moisture content was measured using a Carl Fischer moisture meter (AQV-2200, manufactured by HIRANUMA Co., Ltd.) and an accompanying evaporator (EV-2000, manufactured by HIRANUMA Co., Ltd.) The measurement conditions were a sample amount of 0.1 g, a nitrogen flow rate of 0.2 L/min., and a heating temperature of 150°C.

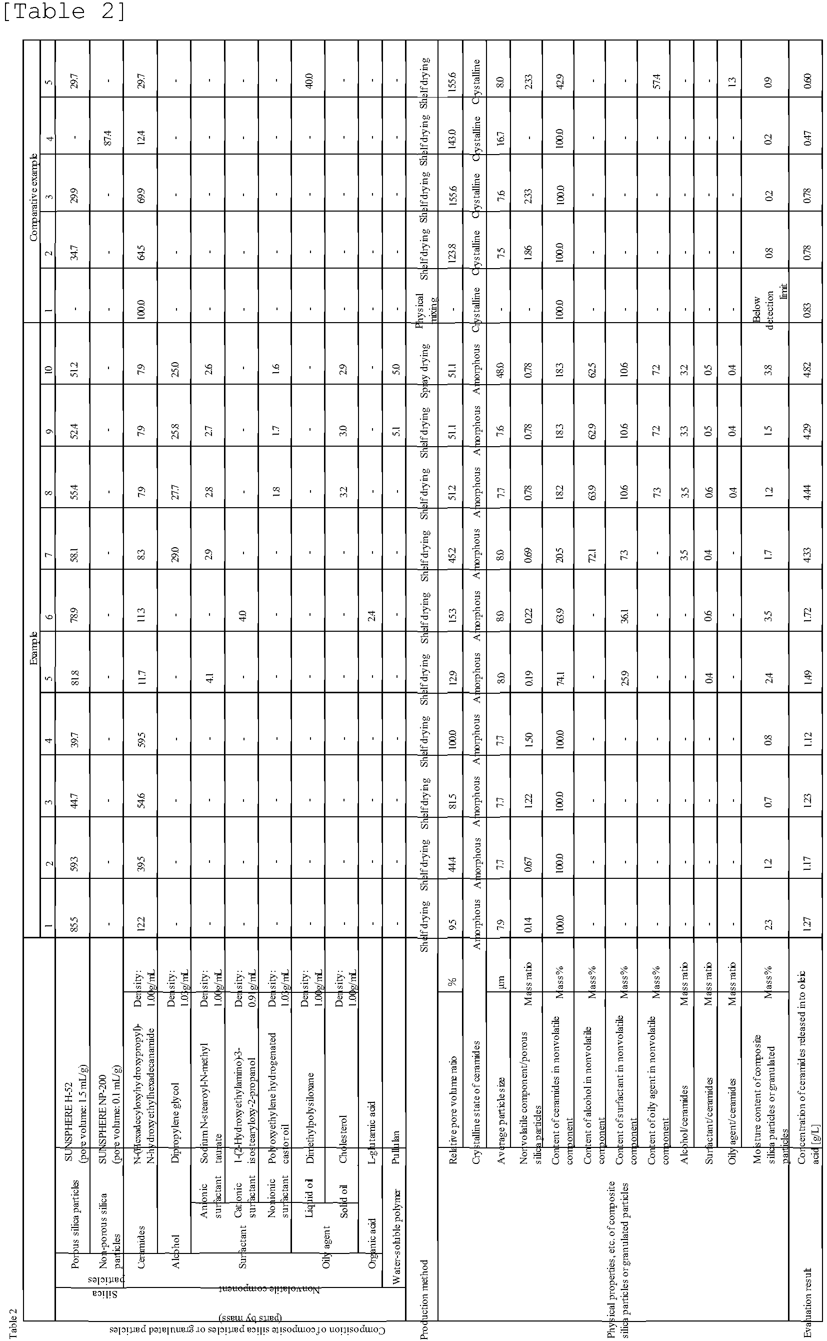

From Table 2, it is confirmed that the composite silica particles of Examples 1 to 9 and the granulated particles of Example 10, which contain porous silica particles and a nonvolatile component containing ceramides, wherein the porous silica particles have a pore volume of 0.5 mL/g or more, the nonvolatile component has a boiling point (at 1,013 hPa) of 150°C or higher, and the relative pore volume ratio is 110% or less, have high concentrations of the ceramides released into oleic acid and can efficiently release ceramides into sebum. As a result, when the particles are used in cosmetic preparations and the like, ceramides are efficiently applied to the skin. Further, since the ceramides in the porous silica particles are in an amorphous state, it is considered that the releasability of ceramides into water such as sweat is also improved.

On the other hand, it is confirmed that Comparative Example 1, which does not contain porous silica particles, Comparative Examples 2, 3, and 5, which have a pore volume occupancy of more than 110%, and Comparative Example 4, which uses non-porous silica particles instead of porous silica particles, have low concentrations of the ceramides released into oleic acid because the ceramides are crystalline.

### Industrial applicability

The present invention can provide composite silica particles and granulated particles containing the composite silica particles and a water-soluble polymer, each of which can readily release ceramides upon contact with oleic acid, a major component of sebum, and methods for producing the same.

## Claims

1. Composite silica particles in which a nonvolatile component containing ceramides is supported on porous silica particles,
wherein:
the porous silica particles have a pore volume of 0.5 mL/g or more;
the nonvolatile component has a boiling point (at normal pressure of 1,013 hPa) of 150°C or higher; and
a relative pore volume ratio determined by the following formula (1) is 110% or less: 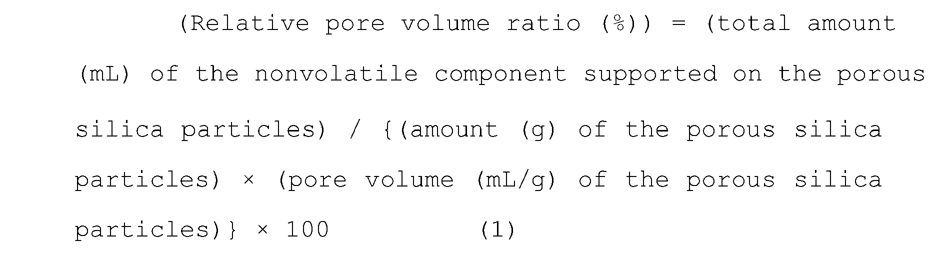 (Relative pore volume ratio (%)) = (total amount (mL) of the nonvolatile component supported on the porous silica particles) / {(amount (g) of the porous silica particles) × (pore volume (mL/g) of the porous silica particles)} × 100 (1)

2. The composite silica particles according to claim 1, wherein the ceramides are in an amorphous state.

3. The composite silica particles according to claim 1 or 2, wherein the mass ratio of the nonvolatile component to the porous silica particles (nonvolatile component/porous silica particles) is 4 or less.

4. The composite silica particles according to any one of claims 1 to 3, wherein:
the nonvolatile component contains an alcohol; and
the alcohol has a solubility in 100 g of water (at 25°C and 1,013 hPa) of more than 1 g.

5. The composite silica particles according to claim 4, wherein the alcohol contains a glycol solvent.

6. The composite silica particles according to claim 4 or 5, wherein the alcohol contains dipropylene glycol.

7. The composite silica particles according to any one of claims 1 to 6, wherein the nonvolatile component contains a surfactant.

8. The composite silica particles according to any one of claims 1 to 7, wherein the content of the ceramides in the nonvolatile component is 5 mass% or more.

9. Granulated particles containing the composite silica particles according to any one of claims 1 to 8.

10. The granulated particles according to claim 9, further containing a water-soluble polymer.

11. The granulated particles according to claim 9 or 10, wherein the granulated particles have a moisture content of 10 mass% or less.

12. A method for producing the composite silica particles according to any one of claims 1 to 8, comprising the following steps 1 and 2 in this order:
step 1: a step of mixing an emulsion or a solution containing the nonvolatile component containing ceramides with the porous silica particles to obtain a suspension; and
step 2: a step of drying the suspension obtained in step 1.

13. A method for producing the granulated particles according to claim 10 or 11, comprising the following steps 1' and 2' in this order:
step 1': a step of mixing an emulsion or a solution containing the nonvolatile component containing ceramides, the porous silica particles, and the water-soluble polymer to obtain a suspension; and
step 2': a step of spray-drying the suspension obtained in step 1'.

14. The method for producing the granulated particles according to claim 13, wherein the water-soluble polymer is a water-soluble polysaccharide.
